# EUROPÄISCHE PATENTSCHRIFT

(11) **EP 2 608 092 B1**
(45) Veröffentlichungstag und Bekanntmachung des Hinweises auf die Patenterteilung: **09.11.2016**
(21) Anmeldenummer: 12197547.8
(22) Anmeldetag: 17.12.2012
(51) Int. Cl.: G06F 19/00, A61B 5/00

(54) **Telediagnosesystem sowie Verfahren zum Durchführen einer Telediagnose**
Remote diagnostic system and method for performing a remote diagnosis
Système de télédiagnostic ainsi que procédé d'exécution d'un télédiagnostic

(30) Priorität: 23.12.2011 DE 102011089793
(43) Veröffentlichungstag der Anmeldung: 26.06.2013
(73) Patentinhaber: Deutsches Zentrum für Luft- und Raumfahrt e.V., 51147 Köln (DE)
(72) Erfinder: Frett, Timo, 53111 Bonn (DE); Poddig, Dirk, 51147 Köln (DE)
(74) Vertreter: dompatent von Kreisler Selting Werner - Partnerschaft von Patent- und Rechtsanwälten mbB

(56) Entgegenhaltungen:
- US-B1- 6 490 490
- YOSHINAGA T ET AL: "Development of 3D space-sharing interface using augmented reality technology for domestic tele-echography", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3. September 2009 (2009-09-03), Seiten 6103-6106, XP031882591, DOI: 10.1109/IEMBS.2009.5334928 ISBN: 978-1-4244-3296-7
- SUENAGA T ET AL: "A tele-instruction system for ultrasound probe operation based on shared ar technology", 2001 CONFERENCE PROCEEDINGS OF THE 23RD ANNUAL INTERNATIONAL CONFERENCE OF THE IEEE ENGINEERING IN MEDICINE AND BIOLOGY SOCIETY (CAT. NO.01CH37272) VOL.3; [ANNUAL INTERNATIONAL CONFERENCE OF THE IEEE ENGINEERING IN MEDICINE AND BIOLOGY SOCIETY. EMBS], Bd. 4, 25. Oktober 2001 (2001-10-25), Seiten 3765-3768, XP010593935, ISBN: 978-0-7803-7211-5

## Beschreibung

Die Erfindung betrifft ein Telediagnosesystem sowie ein Verfahren zum Durchführen einer Telediagnose.

Fachärztliche Diagnostik ist nicht weltweit verfügbar. Um die Qualität der medizinischen Versorgung zu verbessern, ist es z.B. per Videokonferenz möglich, medizinische Expertise über Entfernungen zum Patienten zu übertragen.

Bildgebende medizinische Verfahren können durch Helfer am Patienten eingesetzt werden. Durch den Anschluss der Medizingeräte an ein Videokonferenzsystem können die Bilder von einer Untersuchungsstelle zu einem Facharzt übertragen werden. Muss nun der Bildausschnitt des Medizingerätes verändert werden, wird dieser Wunsch bislang dem Helfer mündlich mitgeteilt oder die Änderung über einen Roboterarm aus der Ferne durch den Facharzt selbst durchgeführt. Hierbei kommt es immer wieder zu Verständigungsproblemen, z.B. wenn der Facharzt relevante Punkte beim Patienten untersuchen will, aber den Helfer vor Ort nicht exakt anleiten kann.

Die Voraussetzung für eine gezielte telemedizinische Diagnose ist die korrekte Darstellung der zu behandelnden Stelle und deren Übertragung zum behandelnden Facharzt.

Die Übertragung von Ultraschallaufnahmen via Telemedizin stellt ein seit vielen Jahren häufig genutztes und bewährtes Verfahren dar (Heese F., Görg C. "Diagnostische Wertigkeit einer internistischen Referenzsonographie (DEGUM-Stufe 3)", Ultraschall in Med 2006; 27(3): 220-224, Georg Thieme Verlag Stuttgart). Die Fernanleitung erfolgt dabei größtenteils per Sprachbefehl. Bei einer mündlichen Anleitung des Helfers gibt der Facharzt Audiokommandos, wie das bildgebende System zu führen ist (Popov V. et al. "The Feasibility of Real-Time Transmission of Sonographic Images from a Remote Location over Low-Bandwidth Internet Links: A Pilot Study", AJR March 2007 vol. 188 no. 3).

Vereinzelt gibt es Versuche, diese Steuerung direkt mit Hilfe eines Roboterarms von einem Facharzt durchführen zu lassen. Hierbei wird der Roboterarm über eine Datenleitung aus der Ferne bedient. Der Helfer vor Ort hat in diesem Fall nur noch unterstützende Funktion und hält den Roboterarm beispielsweise über den Patienten oder überwacht die Untersuchung (Arbeille P., Capri A. et al. "Use of a Robotic Arm to Perform Remote Abdominal Telesonography", American Roentgen Ray Society, AJR: 188, 2007).

Im Bereich der Raumfahrt wurde ein Verfahren erprobt, bei dem zusätzlich zur Sprachanleitung eine Übersichtskarte zur Orientierung verwendet wird. Auf der Karte sind diverse Aufsetzpunkte für den Schallkopf markiert. Ein Astronaut erhält bei diesem Verfahren auf der Erde zunächst ein kurzes Training und soll dann in der Schwerelosigkeit eine Ultraschalluntersuchung mit Hilfe der Übersichtskarte und einer mündlichen Anleitung durchführen (Barratt M, Pool SL "Principles of Clinical Medicine for Space Flight", 2008) (Foale, C.M., Kaleri A.Y., et al. "Diagnostic Instrumentation Aboard ISS: Just-In-Time Training for Non-Physician Crewmembers", Aviation, Space and Environmental Medicine, Vol. 76, No. 6, 2005).

Der genannte Stand der Technik weist die folgenden Nachteile auf: Eine rein mündliche Übermittlung von Anweisungen ist mitunter unpräzise. Ferner müssen häufig Sprachbarrieren überwunden werden. Die Verwendung eines Roboterarmes ist sehr kosten- und wartungsintensiv. Ferner muss der Roboterarm häufig vom Helfer mit bedient werden. Für die Übertragung des Steuerbefehl des Roboterarms sind große Datenraten erforderlich, was eine zusätzliche teure Datenleitung erforderlich macht oder zu einer Reduktion der für die Bildübertragung zur Verfügung stehenden Datenrate führt.

Eine Anleitung mittels Übersichtskarte dient lediglich zur besseren Auffindung der Aufsetzpunkte der Diagnosevorrichtung, beseitigt aber keine Verständigungsschwierigkeiten während der Untersuchung.

Die Druckschrift -YOSHINAGA T ET AL: "Development of 3D space-sharing interface using augmented reality technology for domestic tele-echography", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3. September 2009 (2009-09-03), Seiten 6103-6106, XP031882591, DOI: 10.1109/IEMBS.2009.5334928 ISBN: 978-1-4244-3296-7 - beschreibt ein Verfahren zum Fernbedienen eines Ultraschallgerätes. Es werden Marker am Patientenkörper und an der Ultraschallsonde verwendet, um die Position der Ultraschallsonde gegenüber dem Patientenkörper zu ermitteln. Die Marker werden durche ein USB-Kamera erfasst.

Die Druckschrift - SUENAGA T ET AL: "A tele-instruction system for ultrasound probe operation based on shared ar technology", 2001 CONFERENCE PROCEEDINGS OF THE 23RD ANNUAL INTERNATIONAL CONFERENCE OF THE IEEE ENGINEERING IN MEDICINE AND BIOLOGY SOCIETY (CAT. NO.01CH37272) VOL.3; [ANNUAL INTERNATIONAL CONFERENCE OF THE IEEE ENGINEERING IN MEDICINE AND BIOLOGY SOCIETY. EMBS], Bd. 4, 25. Oktober 2001 (2001-10-25), Seiten 3765-3768, XP010593935, ISBN: 978-0-7803-7211-5- und die Druckschrift US6 490 490 B1 beschreiben ebenfalls ähnliche Telediagnosesysteme.

Aufgabe der Erfindung ist es, ein Telediagnosesystem und ein Verfahren zum Durchführen einer Telediagnose bereitzustellen, die eine einfachere und sicherere Durchführung einer Telediagnose ermöglichen.

Die Lösung der Aufgabe erfolgt erfindungsgemäß durch die Merkmale des Vorrichtungsanspruchs 1 und des Verfahrensanspruchs 6.

Das erfindungsgemäße Telediagnosesystem weist eine Eingabevorrichtung zum Eingeben von Befehlen durch einen ersten Benutzer auf. Diese Befehle betreffen die Bewegung einer räumlich von der Eingabevorrichtung beabstandeten Diagnosevorrichtung. Bei dem ersten Benutzer kann es sich beispielsweise um einen Arzt handeln, so dass die Diagnosevorrichtung als medizinische Diagnosevorrichtung ausgestaltet ist und der Arzt mit Hilfe des Telediagnosesystems einen räumlich entfernten Patienten behandeln kann.

Erfindungsgemäß umfasst das Telediagnosesystem ferner eine Anzeigevorrichtung, die datentechnisch mit der Eingabevorrichtung verbindbar ist. Die Anzeigevorrichtung dient dem Anzeigen der durch den ersten Benutzer angegebenen Bewegungsbefehle an einen von dem ersten Benutzer räumlich beabstandeten zweiten Benutzer. Bei diesem zweiten Benutzer kann es sich beispielsweise um einen nicht besonders ausgebildeten Helfer handeln, der sich bei der zu untersuchenden Person oder dem zu untersuchenden Gegenstand befindet und die Diagnosevorrichtung gemäß den durch den ersten Benutzer eingegebenen Bewegungsbefehlen bewegt. Somit kann die Diagnosevorrichtung genau diejenigen Daten aufnehmen, die der erste Benutzer zum Stellen seiner Telediagnose benötigt. Bei der Anzeigevorrichtung kann es sich beispielsweise um eine optische Anzeigevorrichtung handeln. Ihre Verbindung mit der Eingabevorrichtung kann auch über große Strecken erfolgen, so dass es beispielsweise möglich ist, einen Helfer zum Bewegen der Diagnosevorrichtung anzuleiten, der mehrere Hunderte oder Tausende Kilometer entfernt ist. Trotz dieser großen räumlichen Entfernung kann der erste Benutzer den zweiten Benutzer durch das erfindungsgemäße Telediagnosesystem einfach und dennoch sehr genau anleiten, so dass eine effiziente Diagnose möglich wird.

Es ist erfindungsgemäß vorgesehen, dass die Anzeigevorrichtung derart mit einer Diagnosevorrichtung verbindbar oder verbunden ist, dass die Anzeigevorrichtung und die Diagnosevorrichtung relativ zueinander nicht beweglich sind, wobei die Verbindung dieser beiden Komponenten insbesondere durch einen lösbaren Schnellverschluss erfolgt.

Anders ausgedrückt, wird somit die Anzeigevorrichtung in gleicher Weise mitbewegt, wenn die Diagnosevorrichtung durch den zweiten Benutzer bewegt wird. Die Anzeigevorrichtung hat somit immer exakt dieselbe räumliche Lage und Ausrichtung wie die Diagnosevorrichtung. Bewegungsbefehle, die durch die Anzeigevorrichtung angezeigt werden, können somit vom zweiten Benutzer auf sehr einfache Weise intuitiv umgesetzt werden, da er die Diagnosevorrichtung lediglich in diejenige Richtung bewegen muss, die durch die Anzeigevorrichtung angezeigt wird. Ein Umdenken durch den zweiten Benutzer im Sinne eines Umrechnens in ein anderes Koordinatensystem ist nicht notwendig.

Es ist weiterhin bevorzugt, dass die Eingabevorrichtung einen Drei-Achsen-Joystick aufweist. Dieser dient der Eingabe eines Befehls für eine translatorische Bewegung der Diagnosevorrichtung entlang zweier senkrecht zueinander stehender Achsen und insbesondere einer rotatorischen Bewegung um eine dritte Achse, die ebenfalls senkrecht zu den ersten zwei Achsen steht. Die Anzeigevorrichtung umfasst in dieser Ausführungsform sechs insbesondere optische Anzeigeelemente, nämlich jeweils zwei Anzeigeelemente für jede der genannten drei Achsen (jeweils ein Anzeigeelement für jede Richtung einer Achse). Durch diese Anzeigeelemente erfolgt ein Anzeigen der durch den Drei-Achsen-Joystick eingegebenen Befehle entlang bzw. um die genannten drei Achsen. Die optischen Anzeigeelemente können beispielsweise als gerade Pfeile für die Anzeige der translatorischen Bewegungen und als ringsegmentförmige Pfeile zur Anzeige der rotatorischen Bewegung ausgebildet sein.

Es ist möglich, dass die Eingabevorrichtung weitere Eingabeelemente aufweist. Beispielsweise kann sie einen Schieberegler zur Eingabe eines Befehls für eine translatorische Bewegung der Diagnosevorrichtung entlang der dritten Achse aufweisen. In dieser Ausführungsform weist die Anzeigevorrichtung weitere zwei Anzeigeelemente auf zum Anzeigen der durch den Schieberegler eingegebenen Bewegung entlang dieser dritten Achse. Durch die genannten Eingabe- und Anzeigeelemente des erfindungsgemäßen Telediagnosesystems ist es somit dem ersten Benutzer möglich, Befehle über Bewegungen entlang dreier Achsen und um eine dieser Achsen an den zweiten Benutzer zu übermitteln. Es ergeben sich somit vier Freiheitsgrade. Handelt es sich beispielsweise bei der Diagnosevorrichtung um eine medizinische Diagnosevorrichtung, kann diese unter Verwendung der genannten vier Freiheitsgrad in geeigneter Weise platziert werden. Bei der Diagnosevorrichtung kann es sich beispielsweise um ein Sonographiegerät oder anderes Gerät für ein bildgebendes Verfahren, beispielsweise im Bereich der Dermatologie, handeln. Jedoch kann die Diagnosevorrichtung auch ein nicht medizinisches Gerät sein, durch das eine Untersuchung einer räumlich entfernten Person oder eines räumlich entfernten Gegenstandes erfolgen kann.

Die Eingabevorrichtung und die Anzeigevorrichtung können weitere Eingabeelemente und Anzeigeelemente aufweisen, durch die die Übertragung von Bewegungsbefehlen bezüglich der verbleibenden zwei Freiheitsgrade möglich wird. Diese Bewegungsbefehle betreffen dann eine Rotation der Diagnosevorrichtung jeweils um die erste und zweite Achse. Durch diese zusätzlichen Eingabeelemente kann somit die Diagnosevorrichtung durch die Befehle des ersten Benutzers beliebig im Raum platziert werden.

Zur eigenen Kontrolle der durch den ersten Benutzer eingegebenen Bewegungsbefehle ist es bevorzugt, dass die Eingabevorrichtung eine zweite optische Anzeigevorrichtung zum Anzeigen der durch den ersten Benutzer in die Eingabevorrichtung eingegebenen Befehle aufweist. Diese ist insbesondere identisch zu der ersten Anzeigevorrichtung zum Anzeigen der Befehle an den zweiten Benutzer ausgebildet. Der erste Benutzer sieht somit, anders ausgedrückt, die eingegebenen Bewegungsbefehle in identischer Weise wie der zweite Benutzer. Mögliche Eingabefehler können somit schnell erkannt und korrigiert werden.

Die Erfindung betrifft ferner ein Verfahren zum Durchführen einer Telediagnose. Zunächst erfolgt ein Eingeben von Befehlen in eine Eingabevorrichtung durch einen ersten Benutzer. Diese Befehle betreffen die Bewegung einer von der Eingabevörrichtung räumlich beabstandeten Diagnosevorrichtung. Diese durch den ersten Benutzer eingegebenen Bewegungsbefehle werden mittels einer Anzeigevorrichtung einem zweiten Benutzer, der räumlich vom ersten Benutzer beabstandet ist, angezeigt.

Das erfindungsgemäße Verfahren kann dieselben Merkmale aufweisen, die in Zusammenhang mit dem erfindungsgemäßen Telediagnosesystem beschrieben wurden und umgekehrt.

Es ist bevorzugt, dass die durch den ersten Benutzer in die Eingabevorrichtung eingegebenen Befehle zur Kontrolle durch den ersten Benutzer optisch durch eine zweite Anzeigevorrichtung in der Eingabevorrichtung angezeigt werden.

Das erfindungsgemäße Telediagnosesystem kann beispielsweise mit den modernen technischen Möglichkeiten eines Videokonferenzsystems kombiniert werden, ohne dass aufwändige Modifikationen oder Ergänzungen notwendig sind. Die benötigte zusätzliche Datenrate ist mit maximal 1-2 Kilobit pro Sekunde sehr gering und kann somit ohne eine sichtbare Beeinträchtigung der Bildqualität des übertragenen Diagnosebildes, beispielsweise eines Ultraschallbildes, in der bereits vorhandenen Datenleitung versendet werden. Eine zusätzliche Datenleitung oder -übertragung ist nicht erforderlich.

Gegebenenfalls kann es sinnvoll sein, das Telediagnosesystem mit einer Übersichtskamera, deren Bild ebenfalls mittels Videokonferenz übertragen wird, zu kombinieren, so dass es dem ersten Benutzer möglich ist, die gewünschte Untersuchungsstelle schnell zu erreichen. Die optische Darstellung der gewünschten Bewegungsrichtung der Diagnosevorrichtung erhöht ggf. in Kombination mit einer akustischen Übermittlung von Sprachanweisungen des ersten Benutzers die Genauigkeit und Geschwindigkeit der Telediagnose.

Das erfindungsgemäße Telediagnosesystem kann besonders kostengünstig durch Verwendung von vorhandenen Serienprodukten hergestellt werden. Spezialkomponenten sind nicht erforderlich.

Im Folgenden werden bevorzugte Ausführungsformen der Erfindung anhand von Figuren erläutert.

Es zeigen:
- Figur 1: eine Ausführungsform einer erfindungsgemäßen Anzeigevorrichtung,
- Figur 2: eine Ausführungsform einer erfindungsgemäßen Anzeigevorrichtung,
- Figur 3: eine Ausführungsform einer erfindungsgemäßen Eingabevorrichtung.

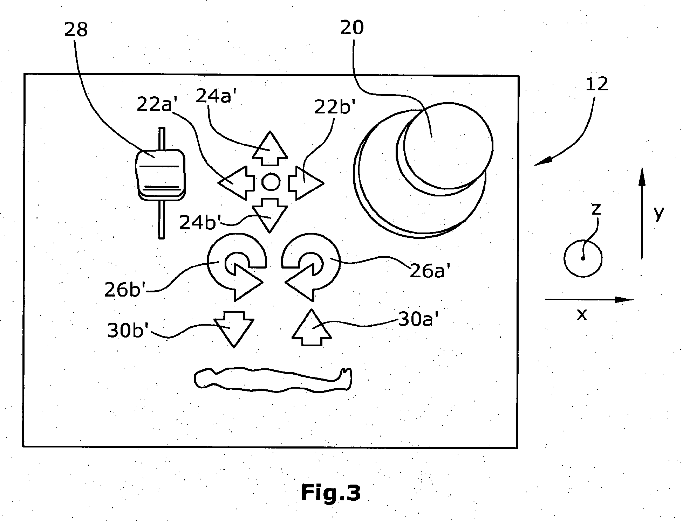

Die in Fig. 3 dargestellte Eingabevorrichtung 12 ist als Steuerpult ausgebildet und weist einen Drei-Achsen-Joystick 20 auf, durch den Befehle für eine translatorische Bewegung der Diagnosevorrichtung 16 entlang zweier Achsen x, y eingegeben werden können. Ferner erzeugt eine Rotation des Joysticks 20 ein Befehl für eine rotatorische Bewegung um eine dritte Achse z.

Zur Kontrolle der durch den Joystick 20 eingegebenen Befehle durch den ersten Benutzer weist das Steuerpult 12 sechs Leuchtelemente auf. Die oben angeordneten vier geraden Pfeile 22a', 22b', 24a', 24b' stellen die Bewegungsbefehle entlang der translatorischen Achsen x und y dar. Die ringsegmentförmigen Pfeile 26a', 26b' stellen die Befehle hinsichtlich der Rotationsbewegungen um die dritte Achse z dar. Bevorzugt sind diese Anzeigeelemente durchsichtig oder durchscheinend ausgebildet, wobei sich unter diesen Anzeigeelementen ein Leuchtmittel, beispielsweise jeweils eine LED befindet, durch die die entsprechende Bewegungsanweisung angezeigt wird.

Das Steuerpult 12 weist ferner einen Schieberegler 28 auf, durch den ein Befehl hinsichtlich einer translatorischen Bewegung entlang der dritten Achse z eingegeben werden kann. Ist beispielsweise die Diagnosevorrichtung 16 ein Ultraschallgerät, so muss dem ersten Benutzer die Möglichkeit gegeben werden, den Druck, mit dem dieses Ultraschallgerät auf die zu untersuchende Stelle gedrückt wird, zu bestimmen. Hierfür stehen die beiden Befehle "Erhöhen des Drucks" und "Verringern des Drucks" zur Verfügung. Diese Befehle werden durch die zusätzlichen Anzeigeelemente 30a', 30b' angezeigt.

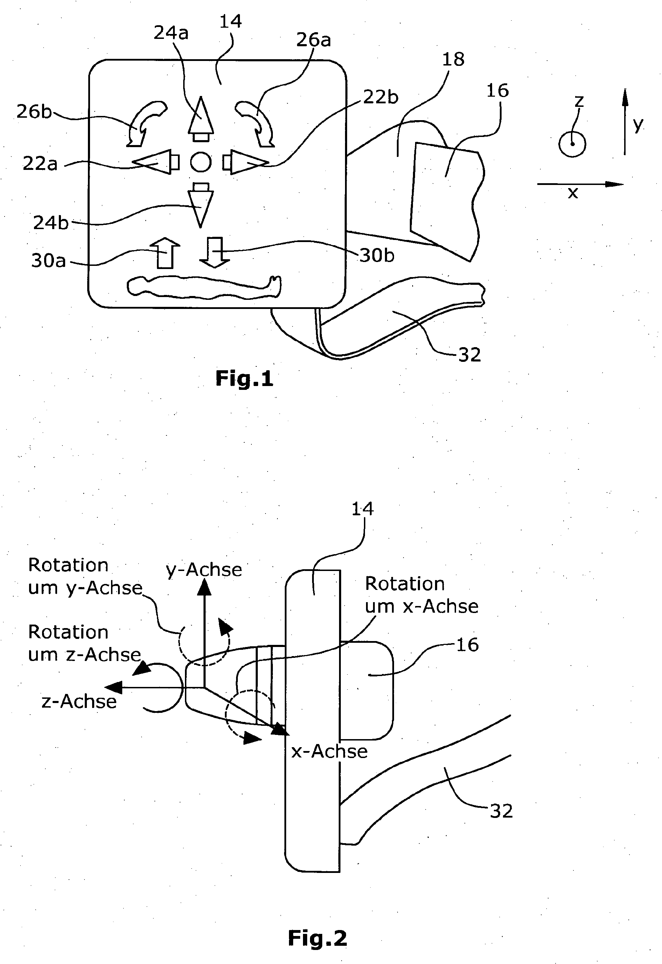

Dieselben Anzeigeelemente finden sich in der Anzeigevorrichtung 14, die in der Fig. 2 dargestellt ist. Diese Anzeigevorrichtung 14 ist vorzugsweise mechanisch in nicht beweglicher Weise mit der Untersuchungsvorrichtung 16, beispielsweise einem Ultraschallkopf, verbunden. Dieser Ultraschallkopf wird durch den zweiten Benutzer gemäß den Bewegungsanweisungen des ersten Benutzers geführt. Der Anzeigevorrichtung 14 kann der zweite Benutzer Bewegungsanweisungen entnehmen, die vier Freiheitsgrade, nämlich drei translatorische Bewegungen entlang der Achsen x, y, z und eine rotatorische Bewegung um die Achse z, betreffen. Die verbleibenden zwei Freiheitsgrade, nämlich die Rotationsbewegung um die Achsen x und y, können ebenfalls durch weitere Eingabeelemente und Anzeigeelemente implementiert werden.

In Fig. 1 ist die Anzeigevorrichtung 14 und die mit dieser verbundene Diagnosevorrichtung 16 dargestellt, wobei diese Komponenten im Verhältnis zu Fig. 2 um 90° senkrecht zur Blattebene nach links geschwenkt wurden. Die Anzeigevorrichtung 14 ist mit einer nicht dargestellten Steuer- und Berechnungseinrichtung verbunden. Die drei Achsen x, y und z, entlang derer eine translatorische Bewegung durchgeführt wird, sind in Fig. 1 ebenfalls gekennzeichnet. Ebenso ist mit dem durchgehenden kreissegmentförmigen Pfeil auf der linken Seite die Rotation um die z-Achse dargestellt. Somit wären die eingangs genannten vier Freiheitsgrade für Bewegungsbefehle vollständig. Die gestrichelt dargestellten Rotationsbefehle um die x- und y-Achse können, wie bereits dargestellt, zusätzlich implementiert werden, so dass es möglich ist, die Diagnosevorrichtung beliebig im Raum anzuordnen. Die bisher verwendete Elektronik für Bewegungsbefehle in vier Freiheitsgraden ist auch dazu in der Lage, sechs Freiheitsgrade zu bedienen, so dass beliebige Bewegungen möglich sind.

Es ist bevorzugt, dass die aktuelle Stellung der Eingabebefehle durch den ersten Benutzer kontinuierlich über einen Mikroprozessor abgefragt und verarbeitet wird. Dieser befindet sich in der Eingabevorrichtung 12 oder kann datentechnisch mit dieser verbunden sein. Der verwendete Mikroprozessor (z.B. M-Unit 2, C-Control) verarbeitet in einem z.B. in Visualbasic++ geschriebenen Programm die eingegebenen Richtungsbefehle. Das Programm wandelt diese Steuerbefehle für die Diagnosevorrichtung um und sendet sie z.B. über eine serielle Verbindung (RS 232) zunächst an den telemedizinischen Arbeitsplatz des ersten Benutzers, der diese Befehle über die Anzeigevorrichtung 14' kontrollieren kann. Über einen Datenkanal, beispielsweise den Datenkanal der Videokonferenz, werden die Richtungsbefehle dann an die Anzeigevorrichtung weitergeleitet, während sie gleichzeitig über eine LED-Anzeige auf dem Eingabepult 12 dargestellt werden. Gemäß Fig. 1 kann die Diagnosevorrichtung 16 in einfacher Weise, beispielsweise mittels eines Klettverschlusses mit der Anzeigevorrichtung 14 verbunden werden.

Die Untersuchungskomponente, die in Fig. 2 dargestellt ist, kann die Anzeigevorrichtung 14 und eine separate, nicht dargestellte, Empfangseinheit aufweisen. Dies kann aus ergonomischen Gründen vorteilhaft sein. Diese beiden Einheiten sind datentechnisch miteinander verbunden. Ein entsprechendes Datenkabel 32 ist beispielsweise in den Fign. 1 und 2 sichtbar. Die Empfangseinheit kann über eine serielle Verbindung mit dem telemedizinischen Arbeitsplatz des ersten Benutzers verbunden werden und kann einen Mikroprozessor (M-Unit 2, C-Control) aufweisen, welcher die Steuerbefehle der Eingabevorrichtung 12 empfängt und mit Hilfe der Anzeigevorrichtung 14 optisch anzeigt. Eine kabelgebundene Verbindung der Anzeigevorrichtung 14 mit der nicht dargestellten Empfangseinheit kann vorteilhaft sein, um eine Steuerung von umliegenden Medizingeräten aufgrund einer Funkübertragung zu vermeiden.

Das erfindungsgemäße Telediagnosesystem sowie das erfindungsgemäße Verfahren können beispielsweise in der Medizintechnik sowohl im Bereich der telemedizinischen Versorgung in abgelegenen Regionen oder Katastrophengebieten (z.B. für Rettungskräfte nach Überschwemmungen oder Erdbeben) eingesetzt werden. Weiterhin ist ein Einsatz als Komponente zur Qualitätssicherung bei Aus- und Weiterbildungsmaßnähmen (Teleteaching, E-Learning) denkbar.

## Patentansprüche

1. Telediagnosesystem (10), mit
einer Eingabevorrichtung (12) zum Eingeben von Befehlen durch einen ersten Benutzer, die die Bewegungen einer räumlich von der Eingabevorrichtung (12) beabstandeten Diagnosevorrichtung (16) betreffen,
einer Anzeigevorrichtung (14), die datentechnisch mit der Eingabevorrichtung (12) verbindbar ist, zum Anzeigen der durch den ersten Benutzer eingegebenen Bewegungsbefehle an einen von diesem räumlich beabstandeten zweiten Benutzer,
**dadurch gekennzeichnet dass**,
die Anzeigevorrichtung (14) derart mit einer Diagnosevorrichtung (16) verbindbar oder verbunden ist, dass die Anzeigevorrichtung (14) und die Diagnosevorrichtung (16) relativ zueinander nicht beweglich sind.

2. Telediagnosesystem (10) nach Anspruch 1, **dadurch gekennzeichnet, dass** die Verbindung zwischen der Anzeigevorrichtung (14) und der Diagnosevorrichtung (16) durch einen lösbaren Schnellverschluss (18) erfolgt.

3. Telediagnosesystem (10) nach Anspruch 1 oder 2, **dadurch gekennzeichnet, dass** die Eingabevorrichtung (12) einen Drei-Achsen-Joystick (20) aufweist zur Eingabe eines Befehls für eine translatorische Bewegung der Diagnosevorrichtung (16) entlang zweier Achsen (x, y) und einer rotatorischen Bewegung um eine dritte Achse (z) und die Anzeigevorrichtung (14) sechs optische Anzeigeelemente (22ab, 24ab, 26ab), nämlich je zwei Anzeigeelemente für jede dieser drei Achsen (x, y, z) aufweist, zum Anzeigen der durch den Drei-Achsen-Joystick (20) eingegebenen Befehle entlang bzw. um die drei Achsen (x, y, z) aufweist.

4. Telediagnosesystem (10) nach einem der Ansprüche 1 bis 3, **dadurch gekennzeichnet, dass** die Eingabevorrichtung (12) einen Schieberegler (28) zur Eingabe eines Befehls für eine translatorische Bewegung der Diagnosevorrichtung (16) entlang der dritten Achse (z) aufweist und die Anzeigevorrichtung (14) zwei Anzeigeelemente (30ab) aufweist zum Anzeigen der durch den Schieberegler (28) eingegebenen Bewegungen entlang der dritten Achse (z).

5. Telediagnosesystem (10) nach einem der Ansprüche 1 bis 4, **dadurch gekennzeichnet, dass** die Eingabevorrichtung (12) eine zweite optische Anzeigevorrichtung (14') zum Anzeigen der durch den ersten Benutzer in die Eingabevorrichtung (12) eingegebenen Befehle aufweist, die insbesondere identisch zu der ersten Anzeigevorrichtung (14) zum Anzeigen der Befehle an den zweiten Benutzer ausgebildet ist.

6. Verfahren zum Durchführen einer Telediagnose, mit den Schritten:
- Eingeben von Befehlen in eine Eingabevorrichtung (12) durch einen ersten Benutzer, wobei die Befehle die Bewegung einer von der Eingabevorrichtung (12) räumlich beabstandeten Diagnosevorrichtung (16) betreffen,
- Anzeigen der durch den ersten Benutzer eingegebenen Bewegungsbefehle mittels einer Anzeigevorrichtung (14) an einen zweiten Benutzer, der auch räumlich vom ersten Benutzer beabstandet ist,
**dadurch gekennzeichnet dass**,
die Anzeigevorrichtung (14) derart mit einer Diagnosevorrichtung (16) verbindbar oder verbunden ist, dass die Anzeigevorrichtung (14) und die Diagnosevorrichtung (16) relativ zueinander nicht beweglich sind.

7. Telediagnosesystem (10) nach Anspruch 6, **dadurch gekennzeichnet, dass** die durch den ersten Benutzer in die Eingabevorrichtung (12) eingegebenen Befehle zur Kontrolle durch den ersten Benutzer optisch und durch eine zweite Anzeigevorrichtung (14') in der Eingabevorrichtung (12) angezeigt werden.

## Claims

1. A remote diagnostic system (10), comprising
an input device (12) for input of instructions by a first user, said instructions pertaining to the movements of a diagnostic device (16) spatially remote from the input device (12),
a display device (14) which is connectible to the input device (12) for data communication, said display device provided to display the movement instructions input by the first user to a second user who is spatially remote from the first user,
**characterized in that**
the display device (14) is connectible or connected to a diagnostic device (16) in such a manner that the display device (14) and the diagnostic device (16) are not movable relative to each other.

2. The remote diagnostic system (10) according to claim 1, **characterized in that** the connection between the display device (14) and the diagnostic device (16) is realized by a releasable quick-acting fastener (18).

3. The remote diagnostic system (10) according to claim 1 or 2, **characterized in that** the input device (12) comprises a three-axis joystick (20) for input of an instruction for a translatory movement of the diagnostic device (16) along two axes (x,y) and a rotary movement about a third axis (z), and that the display device (14) comprises six optical display elements (22ab,24ab,26ab), notably respectively two display elements for each of said three axes (x,y,z), for displaying the instructions input via the three-axis joystick (20) along and respectively about the three axes (x,y,z).

4. The remote diagnostic system (10) according to any one of claims 1 to 3, **characterized in that** the input device (12) comprises a slide controller (28) for input of an instruction for a translatory movement of the diagnostic device (16) along the third axis (z), and that the display device (14) comprises two display elements (30ab) for display of the movements along the third axis (z) as input via the slide controller (28).

5. The remote diagnostic system (10) according to any one of claims 1 to 4, **characterized in that** the input device (12) comprises a second optical display device (14') for display of the instructions input into the input device (12) by the first user, said second optical display device being preferably identical in design to said first display device (14) for display of the instructions to the second user.

6. A method for performing a remote diagnosis, comprising the steps of:
- inputting instructions into an input device (12) by a first user, said instructions pertaining to the movements of a diagnostic device (16) spatially remote from the input device (12),
- by means of a display device (14), displaying the movement instructions input by the first user to a second user who is also spatially remote from the first user,
**characterized in that**
the display device (14) is connectible or connected to a diagnostic device (16) in such a manner that the display device (14) and the diagnostic device (16) are not movable relative to each other.

7. The remote diagnosis system (10) according to claim 6, **characterized in that** the instructions input into the input device (12) by the first user are displayed, for control by the first user, optically and by a second display device (14') in the input device (12).

## Revendications

1. Système de télédiagnostic (10) avec
un dispositif d'entrée (12) pour l'introduction de commandes par un premier utilisateur qui concernent les déplacements d'un dispositif de diagnostic (16) se trouvant à distance du dispositif d'entrée (12), un dispositif d'affichage (14) qui est susceptible d'être relié par voie de données au dispositif d'entrée (12), pour l'affichage des commandes de déplacement introduites par le premier utilisateur, pour un second utilisateur espacé de ce dernier,
**caractérisé en ce que**
le dispositif d'affichage (14) est apte à être relié ou est relié à un dispositif de diagnostic (16) de façon telle que le dispositif d'affichage (14) et le dispositif de diagnostic (16) ne soient pas déplaçables l'un par rapport à l'autre.

2. Système de télédiagnostic (10) selon la revendication 1, **caractérisé en ce que** la liaison entre le dispositif d'affichage (14) et le dispositif de diagnostic (16) est réalisée par une attache rapide (18) détachable.

3. Système de télédiagnostic (10) selon la revendication 1 ou 2, **caractérisé en ce que** le dispositif d'entrée (12) comprend une manette de jeu à trois axes (20) pour l'introduction d'une commande pour un déplacement en translation du dispositif de diagnostic (16) le long de deux axes (x, y) et d'un mouvement de rotation autour d'un troisième axe (z) et que le dispositif d'affichage (14) comprend six éléments d'affichage optiques (22ab, 24ab, 26ab), à savoir deux éléments d'affichage pour chacun de ces trois axes (x, y, z), pour l'affichage des commandes introduites par la manette de jeu à trois axes (20) le long ou autour des trois axes (x, y, z).

4. Système de télédiagnostic (10) selon l'une des revendications 1 à 3, **caractérisé en ce que** le dispositif d'entrée (12) comprend un curseur (28) pour l'introduction d'une commande pour un déplacement en translation du dispositif de diagnostic (16) le long du troisième axe (z) et que le dispositif d'affichage (14) comprend deux éléments d'affichage (30ab) pour l'affichage des déplacements le long du troisième axe (z) introduits par le curseur (28).

5. Système de télédiagnostic (10) selon l'une des revendications 1 à 4, **caractérisé en ce que** le dispositif d'entrée (12) comprend un second dispositif d'affichage (14') pour l'affichage des commandes de déplacement introduites dans le dispositif d'entrée (12) par le premier utilisateur, qui est notamment identique au premier dispositif d'affichage (14) pour l'affichage des commandes pour le second utilisateur.

6. Procédé d'exécution d'un télédiagnostic avec les étapes :
- introduction de commandes dans un dispositif d'entrée (12) par un premier utilisateur, les commandes concernant le déplacement d'un dispositif de diagnostic (16) se trouvant à distance du dispositif d'entrée (12),
- affichage des commandes de déplacement introduites par le premier utilisateur, par un dispositif d'affichage (14) pour un second utilisateur également espacé du premier utilisateur,
**caractérisé en ce que**
le dispositif d'affichage (14) est apte à être relié ou est relié à un dispositif de diagnostic (16) de façon telle que le dispositif d'affichage (14) et le dispositif de diagnostic (16) ne soient pas déplaçables l'un par rapport à l'autre.

7. Système de télédiagnostic (10) selon la revendication 6, **caractérisé en ce que** les commandes introduites dans le dispositif d'entrée (12) par le premier utilisateur sont affichées, pour un contrôle par le premier utilisateur, optiquement et par un second dispositif d'affichage (14') dans le dispositif d'entrée (12).
